# EUROPÄISCHE PATENTSCHRIFT

(11) **EP 0 471 161 B1**
(45) Veröffentlichungstag und Bekanntmachung des Hinweises auf die Patenterteilung: **31.08.1994**
(21) Anmeldenummer: 91109613.9
(22) Anmeldetag: 12.06.1991
(51) Int. Cl.: A61K 31/21, A61K 9/12, A61K 9/72, A61K 47/10

(54) **Nitroglycerinhaltiger, hydrophiler, wässriger Pumpspray**
Spray container containing nitroglycerin in a hydrophilic aqueous composition
Atomiseur pharmaceutique contenant nitroglycérine à base d'une composition hydrophilique, aqueuse

(30) Priorität: 17.08.1990 DE 4026072
(43) Veröffentlichungstag der Anmeldung: 19.02.1992
(73) Patentinhaber: SCHWARZ PHARMA AG, D-40789 Monheim (DE)
(72) Erfinder: Klokkers-Bethke, Karin, Dr., W-6074 Rödermark (DE); Münch, Ulrich, Dr., W-4019 Monheim/Rhld. (DE)

(56) Entgegenhaltungen:
- EP-A- 0 241 847
- EP-A- 0 310 910
- FR-A- 2 633 933

## Beschreibung

Die Erfindung betrifft einen nitroglycerinhaltigen, treibgasfreien,hydrophilen, wässrigen Pumpspray und dessen Herstellung.

Nitroglycerin, auch als Glyceroltrinitrat (GTN) bezeichnet, ist ein Wirkstoff zur Behandlung von Angina-pectoris-Anfällen. Er wird u. a. in Notfallsituationen eingesetzt, in denen die Arzneiform einen schnellen Wirkungseintritt ermöglichen muß.

Die in dieser speziellen Indikation eingesetzten Arzneiformen wie Sublingualtabletten oder Zerbeißkapseln besitzen Nachteile. Nachteilig ist u. a., daß in diesen Arzneiformen der Wirkstoff nach der Einnahme erst freigesetzt und verteilt werden muß, bevor er gelöst zur Resorption zur Verfügung steht. Weiterhin tritt unnötiger Zeitverlust bei der akuten Anfallsbehandlung ein, wenn die Arzneiform erst aus der Packung und Blister entnommen werden muß.

Zur Vermeidung der Nachteile dieser Arzneiformen wurden insbesondere treibgashaltige GTN enthaltende Sprays entwickelt. Durch Aufsprühen der wirkstoffhaltigen Dosis in den Mund sollte ein direktes und schnelles Aufbringen einer Lösung des Wirkstoffes auf einen möglichst großen Teil der den Wirkstoff GTN gut resorbierenden Mundschleimhaut gewährleistet werden. Hierdurch soll eine große Fläche erreicht werden, die die Diffusion des Wirkstoffes beschleunigt.

Treibgas enthaltende Sprays besitzen jedoch zahlreiche Nachteile. Die als Treibgas verwendeten niederen Halogenalkane besitzen umweltschädigende Wirkungen. In der Verwendung von Fluorchlorkohlenwasserstoffen und der Verminderung des Ozongehaltes der oberen Luftschichten werden Zusammenhänge formuliert. Viele Länder schränken daher deren Verwendung als Treibgas ein.

Hinsichtlich des Anwendungsgebietes, GTN-haltige Treibgassprays bei der akuten Anfallsbehandlung der Angina-pectoris einzusetzen, weisen die verwendeten Treibgase den Nachteil auf, austrocknende Wirkung zu haben. Da jedoch bei einem Angina-pectoris-Anfall der Mund ohnehin austrocknet, ist die austrocknende Wirkung der Treibgase besonders nachteilig.

Aufgrund der substanzspezifischen Eigenschaften des GTN werden viele nitroglycerinhaltige Präparate beschrieben, die ein Trägerstoffsystem auf der Basis von Fetten und Ölen enthalten.

Hierbei ist jedoch nachteilig, daß diese Präparate mindestens ein Konservierungsmittel enthalten müssen, damit die in den Triglyceriden enthaltenen Fettsäuren nicht zersetzt, oxidiert oder ranzig werden. Derartige Zusätze in Arzneimitteln sind jedoch nicht nur per se unerwünscht, sondern auch hinsichtlich ihrer Fähigkeit, Allergien auslösen zu können.

Weiterhin verhindern lipophile Lösungsmittel, daß der Wirkstoff GTN mit der bei Akutanfällen der Angina pectoris erwünschten Schnelligkeit in die hydrophile Mucosa verteilt wird.

In der Vergangenheit wurde zur Erhöhung der Wirkstoffverfügbarkeit bei treibgashaltigen Dosieraerosolen die Menge der eingesetzten lipophilen Lösungsmittel vermindert. Jedoch wurde die Anflutdauer, meßbar über die maximale Plasmakonzentration (Cₘₐₓ) und Zeitpunkt der maximalen Konzentration (tₘₐₓ), nur geringfügig beeinflußt.

P.M. Dewland et al. [in Herz & Gefäße, 1, 536 - 544 (1987)] erhielten zwar für drei mit lipophilen Lösungen hergestellte GTN-Sprays mit abnehmender Menge der lipophilen Rezepturanteile höhere Cₘₐₓ-Werte (Tab. 1 a.a.O.), jedoch ist tₘₐₓ nicht signifikant unterschiedlich.

Ein anderer Ansatzpunkt, die Verfügbarkeit des Wirkstoffes zu erhöhen, wird in DE 32 46 081 erläutert.

Bei der in diesem Dokument beschriebenen Formulierung wird der Treibgasanteil auf 60 - 95 % der Rezepturbestandteile erhöht.

Der erhöhte Treibgasanteil bewirkt hierbei eine höhere Konzentration des Wirkstoffes im nichtflüchtigen, öligen Lösemittel.

Der Arzneistoff muß jedoch weiterhin erst aus der öligen Wirkstofflösung in die Mucosa diffundieren. Die im Anfallsgeschehen wichtige Anflutung des Wirkstoffes kann dadurch jedoch nicht bedeutend verkürzt werden. Aus Gesichtspunkten gesteigerten Umweltbewußtseins ist ein erhöhter Treibgasanteil nachteilig und daher zu vermeiden.

Eine qualitativ deutliche Verbesserung von Sprays im Anfallsgeschehen ist unter Beibehaltung lipophiler Lösungsmittel in treibgashaltigen Dosieraerosolen nicht möglich.

Ein weiterer Ansatzpunkt, den Wirkstoff schnell in die hydrophile Mucosa zu verteilen, ist die Verwendung eines Lösemittels, das sich mit der wäßrigen Mucosa mischt. Die Sprayformulierung muß hierbei berücksichtigen, das dieses Lösemittel den Wirkstoff ausreichend phlegmatisiert und auch technisch unter Produktionsbedingungen sicher handzuhaben ist.

US- 3, 155, 574 beschreibt eine treibgashaltige Nitroglycerinsprayformulierung zur Inhalation auf hydrophiler Lösemittelbasis, enthaltend den Wirkstoff GTN, 1,2-Propandiol und wasserfreies Ethanol.

Die Inhalation ist unnötig, da GTN ausreichend über die Mundschleimhaut resorbiert wird, was ebenso wie die Resorption durch Reduzierung des First Pass die Bioverfügbarkeit erhöht. Im weiteren ist es für den Patienten im Anfallsgeschehen äußerst schwer, zu inhalieren.

Untersuchungen von H. Laufen et al. in Therapiewoche 34, 963 - 970 (1984) brachten das Ergebnis, daß im Falle einer hydrophilen Rezeptur im Vergleich zu GTN-Sprays auf lipophiler Basis sowohl das Erscheinen des Wirkstoffes im Blut als auch die Menge der resorbierten Substanz schneller bzw. größer ist. Die Zusammensetzung der Zubereitung und das Dosiersystem wurden jedoch nicht publiziert.

EP 0 310 910 beschreibt nun eine hydrophile GTN-Spray-Formulierung, die frei ist von Fluorchlorkohlenwasserstoffen und die außer dem Wirkstoff lediglich Ethanol und Wasser als Lösemittel enthält.

Nachteilig ist bei dieser Formulierung die fehlende Phlegmatisierung des GTN. Verdunstet Lösemittel, wobei besonders der Ethanolgehalt reduziert wird, so fällt GTN als ölige Phase in Form von Tropfen am Boden der Flasche aus. Gleichzeitig bedingt obige Formulierung zwingend den Einsatz von GTN gelöst in Ethanol mit dem Nachteil der Brennbarkeit und Gefährlichkeit dieses Rohstoffes.

DE-OS 39 22 650 beschreibt als Gegenstand der Erfindung ein nitroglycerinhaltiges, treibgasfreies Aerosolpräparat, bestehend aus dem Wirkstoff GTN und 51 - 90 % ein oder mehr aliphatischen Alkoholen mit 2 - 4 Kohlenstoffatomen und 10 - 49 % Polyalkylenglykolen oder 1 - 3-wertigen, 2 - 8 Kohlenstoffatome aufweisenden Alkoholen. Das verwendete Dosiersystem wurde nicht angegeben.

Bei dieser Formulierung ist weiterhin der hohe Gehalt von ein oder mehr aliphatischen Alkoholen mit 2-4 Kohlenstoffatomen nachteilig. Der Verdunstungsgrad des Alkohols und die Flüchtigkeit der Rezepturbestandteile aus handelsüblichen Dosierpumpen ist hoch, so daß nach längerem Nichtbenutzen des Sprays sich die Zusammensetzung ändert und eine nicht der im Vorratsgefäß ursprünglich vorhandenen pharmazeutischen Formulierung entsprechende Zusammensetzung im Anfallsgeschehen zur Anwendung kommt. Schließlich ist der hohe Alkoholanteil auch aus wirtschaftlichen Gesichtspunkten nachteilig, da Alkohol ein teurer Rohstoff ist.

In der Formulierung werden auch bis zu 49 % Polyalkylenglykole verwendet. In pharmazeutischen Zubereitungen sollte jedoch, wann immer möglich, auf polymere Trägerstoffe verzichtet werden, da sie Verunreinigungen wie Initiatormoleküle, Di- und Tripolymere sowie Katalysatorrückstände enthalten. Diese Verunreinigungen sind in ihrer physiologischen Wirkung nicht unbedenklich.

Weiterhin weisen die genannten GTN enthaltenden Sprayformulierungen des Standes der Technik den Nachteil auf, uneinheitliche Mengen und Wirkstoffkonzentrationen pro Sprühstoß auszubringen.

Der Erfindung liegt die Aufgabe zugrunde, unter Behebung der Nachteile des Standes der Technik einen nitroglycerinhaltigen, hydrophilen, wässrigen Pumpspray, der keine Treibgase enthält und einen therapeutisch wirksamen Blutspiegel in kürzestmöglicher Zeit auch nach langer Standzeit oder wiederholter Anwendung gewährleistet sowie ein Verfahren zu dessen Herstellung zu schaffen.

Überraschend wurde nun gefunden, daß eine Sprayzubereitung der erfindungsgemäßen Zusammensetzung, bestehend aus 0,15-0,50 Gew.-% Glyceroltrinitrat, 24,50-24,85 Gew.-% Ethanol, 32,00 Gew.-% Propylenglykol und 43,00 Gew.-% gereinigtes Wasser, eingestellt auf pH 3-6, ein für die Behandlung des Anfallsgeschehens der Angina-pectoris äußerst günstiges pharmakokinetisches Verhalten mit schneller Anflutung und hoher Bioverfügbarkeit aufweist, wobei die Zubereitung chemisch äußerst stabil ist. Auch nach langer Standzeit oder wiederholten in kurzen Zeitabständen erfolgender Anwendung wird eine der pharmazeutischen Zubereitung entsprechende Zusammensetzung gewährleistet. Durch ihren definierten Gehalt an Alkoholen muß die Zusammensetzung trotz des Wassers nicht konserviert werden.

Aus Gründen zunehmender Allergisierungen stellt dies einen weiteren Vorteil dieser Formulierung dar.

Vorteilhaft enthält die Sprayzubereitung 0,30 Gew.-% Nitroglycerin, 24,70 Gew.-% Ethanol, 32,00 Gew.-% 1.2-Propylenglykol und 43,00 Gew.-% gereinigtes Wasser, auf den pH-Wert 6,0 eingestellt.

Vorzugsweise enthält die Sprayzubereitung 0,40 Gew.-% Nitroglycerin, 24,60 Gew.-% Ethanol, 32,00 Gew.-% 1.2-Propylenglykol und 43,00 Gew.-% gereinigtes Wasser, auf den pH-Wert 6,0 eingestellt.

Die erfindungsgemäße Zubereitung kann auf Grund ihres durch die erfindungsgemäße Zusammensetzung ihrer Bestandteile definierten relativ niedrigen Dampfdruckes mit Pump-Zerstäuberdosiersystemen eingesetzt werden. Dabei wird sichergestellt, daß der von den FCKW-haltigen Nitroglycerinsprays an sich bekannte Vorteil wie die unabhängig von der Standzeit nach dem letzten Gebrauch zuverlässige Dosierung nicht verloren geht.

Eine Ausführungsform des erfindungsgemäßen Sprays ist auf den pH-Wert 6 eingestellt und weist die folgende Zusammensetzung auf:

| | |
|---|---|
| Glyceroltrinitrat | 0,30 % |
| Ethanol | 24,70 % |
| 1.2-Propylenglykol | 32,00 % |
| gereinigtes Wasser | 43,00 % |

Diese Zubereitung erfüllt auf Grund ihrer qualitativen und quantitativen Zusammensetzung der Mischungen die Qualitätsanforderungen an das Arzneimittel. Auch bei üblichen Wintertemperaturen kommt es nicht zur Abscheidung von Glyceroltrinitrat in der Kälte.

Die erfindungsgemäße Sprayformulierung kann mittels der in der Arzneimittelherstellung üblichen Verfahren und Technologien hergestellt werden.

Gegenstand der Erfindung ist daher auch ein Verfahren zur Herstellung der erfindungsgemäßen Sprayformulierung, welches dadurch gekennzeichnet ist, daß in an sich bekannter Weise 0,15-0,50 Gew.-% Glyceroltrinitrat, 24,50-24,85 Gew.-% Ethanol, 32,00 Gew.-% 1.2-Propylenglykol sowie 43,00 Gew.-% gereinigtes Wasser miteinander vermischt werden und die erhaltene Lösung in entsprechende mit Sprühköpfen versehene Dosierpumpen enthaltende Primärpackmittel abgefüllt wird.

Nach einer zweckmäßigen Ausführungsform des erfindungsgemäßen Verfahrens werden 0,30 Gew.-% Nitroglycerin, 24,70 Gew.-% Ethanol, 32,00 Gew.-% 1.2-Propylenglykol und 43,00 Gew.-% gereinigtes Wasser gemischt, die erhaltene Lösung auf den pH-Wert 6,0 eingestellt und in geeignete Primärpackmittel verbracht.

Nach einer weiteren vorteilhaften Ausführungsform des erfindungsgemäßen Verfahrens werden 0,40 Gew.-% Nitroglycerin, 24,60 Gew.-% Ethanol, 32,00 Gew.-% 1.2-Propylenglykol und 43,00 Gew.-% gereinigtes Wasser gemischt, die erhaltene Lösung auf den pH-Wert 6,0 eingestellt und in geeignete Primärpackmittel verbracht.

### Herstellung

### Ausführungsbeispiel 1

a) 0,600 g einer propylenglykolhaltigen Glyceroltrinitratlösung, in der Zusammensetzung 30 mg Glyceroltrinitrat und 570 mg 1.2-Propylenglykol, wurden mit 2,50 g Ethanol, 2,630 g 1.2-Propylenglykol und 4,300 g gereinigtem Wasser gemischt und bis zur Homogenität gerührt. Anschließend wurde 0,1 n Salzsäure zugetropft, bis der pH-Wert 6 eingestellt war.
   Die erhaltene Lösung wurde durch ein PVDF-Filter (Porengröße 0,22 µm) der Firma Millipore filtriert. Dann wurde die Lösung in eine Braunglasflasche gefüllt, auf die eine Dosierpumpe mit Sprühkopf aufgeschraubt wurde.
b) Alternativ kann die Lösung in eine Aerosolglasflasche gefüllt werden, auf die eine Dosierpumpe mit Sprühkopf aufgecrimpt wird.

### Ausführungsbeispiel 2

a) 0,600 g einer alkoholischen Glyceroltrinitratlösung, in der Zusammensetzung 30 mg Glyceroltrinitrat und 570 mg Ethanol, wurden mit 1,900 g Ethanol (Merck), 3,200 g 1.2-Propylenglykol und 4,300 g gereinigtem Wasser gemischt und bis zur Homogenität gerührt. Anschließend wurde 0,1 n Salzsäure zugetropft, bis der pH-Wert 6 eingestellt war. Die erhaltene Lösung wurde durch ein PVDF-Filter (Porengröße 0,22 µm) der Firma Millipore filtriert. Dann wurde die Lösung in eine Braunglasflasche gefüllt, auf die eine Dosierpumpe mit Sprühkopf aufgeschraubt wurde.
b) Alternativ kann die Lösung in eine Aerosolglasflasche gefüllt werden, auf die eine Dosierpumpe mit Sprühkopf aufgecrimpt wird.

### Ausführungsbeispiel 3

a) 0,800 g einer alkoholischen Glyceroltrinitratlösung, in der Zusammensetzung 40 mg Glyceroltrinitrat und 760 mg wasserfreies Ethanol, wurden mit 1,700 g Ethanol (Merck), 3,200 g 1.2-Propylenglykol und 4,300 g gereinigtem Wasser gemischt und bis zur Homogenität gerührt. Anschließend wurde 0,1 n Salzsäure zugetropft, bis der pH-Wert 6 eingestellt war. Die erhaltene Lösung wurde durch ein PVDF-Filter (Porengröße 0,22 µm) der Firma Millipore filtriert. Dann wurde die Lösung in eine Braunglasflasche gefüllt, auf die eine Dosierpumpe mit Sprühkopf aufgeschraubt wurde.
b) Alternativ kann die Lösung in eine Aerosolglasflasche gefüllt werden, auf die eine Dosierpumpe mit Sprühkopf aufgecrimpt wird.

Das erfindungsgemäße Pumpspray hat folgende Vorteile:
- es ist umweltfreundlich, da es keine Treibgase enthält,
- beim Anwender werden keine Allergien ausgelöst, da es frei von Konservierungsstoffen ist,
- es enthält keine polymeren Bestandteile, so daß keine Initiatormoleküle oder Katalysatorrückstände vorhanden sind, deren physiologische Wirkung nicht unbedenklich ist,
- es weist eine hohe Lagerbeständigkeit auf,
- nach längerer Standzeit oder mehrfach aufeinander folgender Anwendung ist eine gleichbleibende Ausbringmenge in gleichbleibender Zusammensetzung gewährleistet.

Die positiven Eigenschaften des erfindungsgemäßen Pumpsprays wurden durch pharmakokinetische Untersuchungen bestätigt.

In einer Bioverfügbarkeitsstudie wurde an 15 Probanden im randomisierten Cross over jeweils 0,10 ml des erfindungsgemäßen Spray A, der wasserfreien Sprayzubereitung B sowie eine handelsübliche Sublingualtablette gleicher Dosis appliziert und 1,2,3,4,5,6,7,10,20,30 Min. nach Applikation die Blutspiegel bestimmt. Die handelsübliche Sublingualtablette Nitropen® bestand aus 0,3 mg GTN und üblichen Tablettenhilfsstoffen. Der wasserfreie Spray (B) hatte die Zusammensetzung 0,377 Gew.-% Glyceroltrinitrat, 89,623 Gew.-% Ethanol und 10,0 Gew.-% 1.2-Propylenglykol. Der Spray (A) weist die gemäß dem Ausführungsbeispiel 2 beschriebene Zusammensetzung auf.

Das Ergebnis der Bioverfügbarkeitsstudie ist in Tabelle 1 aufgelistet:

**Tab. 1**

| Pharmakokinetische Kenndaten | | | |
|---|---|---|---|
| | AUC [mg/ml] | Cₘₐₓ [mg/ml] | tₘₐₓ [Min] |
| Sublingualtablette | 23,408 | 3,053 | 5,667 |
| erfindungsgemäßer Spray (A) | 24,797 | 2,938 | 3,667 |
| wasserfreier Spray (B) | 22,601 | 3,633 | 4,333 |

In der pharmakokinetischen Studie erreicht die kürzeste Anflutzeit (kürzestes tₘₐₓ) der erfindungsgemäße Spray A, während die Sublingualtablette die langsamste Anflutung und das längste tₘₐₓ aufweist. Der nicht wasserhaltige Spray B liegt zwischen beiden Formulierungen.

Das Ergebnis dieser Studie zeigt, daß die Anflutgeschwindigkeit doch in überraschender Weise über die Lösungseigenschaften der Rezeptur für den Wirkstoff maßgeblich beeinflußbar ist, so daß für den Patienten im Anfallsgeschehen eine schnelle Schmerzbefreiung erreicht wird.

Überraschenderweise erwies sich die erfindungsgemäße Formulierung A in den Funktionsprüfungen mit handelsüblichen Pumpdosierzerstäubern der Formulierung B überlegen:

| Lagerposition des Sprays | Verdunstungsverluste innerhalb von 50 Tagen bei Raumtemperatur | |
|---|---|---|
| | erfindungsgemäßer Spray A [mg] | Vergleichspray B [mg] |
| aufrecht | 3,6 ± 3,7 | 30,6 ± 26,1 |
| liegend | 12,2 ± 6,8 | 118,5 ± 37,6 |
| auf dem Kopf | 12,1 ± 7,5 | 58,9 ± 30,1 |

Der erfindungsgemäße Spray A führt hochgerechnet in der für ein Arzneimittel üblichen Haltbarkeitsdauer von 3 Jahren in der ungünstigsten Lagerposition zu einem unerheblichen Gewichtsverlust von ca. 270 mg. Der Vergleichsspray B dagegen kann 2,6 g Gewichtsverlust in 3 Jahren erleiden, was die Haltbarkeitsdauer des Arzneimittels verkürzt.

Bei einer Füllmenge von ca. 10 - 20 g Lösung/Spraybehälter ist eine ausreichende Stabilität des Arzneimittels in qualitativer und quantitativer Hinsicht nur mit der erfindungsgemäßen Lösung A erreichbar.

An die Pumpdosierzerstäuber von Lösungen für eine vitale Indikation wie die Behandlung des Angina-pectoris-Anfalles sind besondere Anforderungen zu stellen: Im Gegensatz zu beispielsweise Desinfektionsmitteln oder Ähnlichem muß die Verfügbarkeit der Dosis auch nach einer Pause nach der letzten Spraybenutzung gewährleistet werden. Die meisten handelsüblichen Dosierpumpen müssen nach einer Standzeit von z.T. nur 1 Tag mehrfach gepumpt werden, da der Inhalt der Dosierkammer nicht abgeschlossen ist und ungehindert verdunstet. Mit einer Dosierpumpe, die konstruktionsmäßig den Inhalt der Dosierkammer abschließt, kann ein Anpumpen entfallen.

Der Anspruch, unabhängig von der Dauer der Nichtbenutzung des Sprays nach der letzten Anwendung, die volle Dosis im ersten Sprühstoß zu applizieren, ist mit der erfindungsgemäßen Lösung A erreichbar: Denn trotz eines Dosierkammerverschlusses können je nach Oberflächenspannung der zu dosierenden Lösung und in Abhängigkeit ihres Dampfdruckes und ihrer quellenden bzw. entquellenden Wirkung auf die Pumpenteile Verdunstungsverluste auftreten. Dies gilt in besonderem Maße für herkömmliche Sprayformulierungen.

Eine vergleichende Untersuchung zwischen der erfindungsgemäßen Sprayformulierung A und herkömmlichen Sprayformulierungen belegte, daß mit der erfindungsgemäßen Sprayformulierung A selbst nach 36 Tagen der Nichtbenutzung der erforderliche Dosisbereich im 1. Sprühstoß erreicht wurde. Er beträgt 81,8 % ± 6,8 % der mittleren Ausbringmenge, ermittelt an zwölf (12) Pumpsprays.

## Patentansprüche

1. Nitroglycerin und ein oder mehr übliche Hilfsstoffe enthaltende, hydrophile, wässrige Pumpsprayzubereitung, insbesondere mit einem Gehalt an Ethanol, dadurch gekennzeichnet, daß die Zubereitung 0,15 bis 0,50 Gew.-% Nitroglycerin, 24,50 bis 24,85 Gew.-% Ethanol, 32,00 Gew.-% 1.2-Propylenglykol und 43,00 Gew.-% gereinigtes Wasser enthält und auf pH 3 bis 6 eingestellt ist.

2. Pumpsprayzubereitung nach Anspruch 1, dadurch gekennzeichnet, daß die Zubereitung 0,30 bis 0,40 Gew.-% Nitroglycerin und 24,60 bis 24,70 Gew.-% Ethanol enthält und auf den pH-Wert 6,0 eingestellt ist.

3. Verfahren zur Herstellung einer Nitroglycerin und ein oder mehr übliche Hilfsstoffe enthaltenden, hydrophilen, wässrigen Pumpsprayzubereitung nach Ansprüchen 1 und 2, dadurch gekennzeichnet, daß man in an sich bekannter Weise 0,15 bis 0,50 Gew.-% Nitroglycerin, 24,50 bis 24,85 Gew.-% Ethanol, 32,00 Gew.-% 1.2-Propylenglykol und 43,00 Gew.-% gereinigtes Wasser mischt, die erhaltene Lösung auf pH 3-6 einstellt und in geeignete Primärpackmittel verbringt.

4. Verfahren zur Herstellung einer Nitroglycerin und ein oder mehr übliche Hilfsstoffe enthaltenden, hydrophilen, wässrigen Pumpsprayzubereitung nach Anspruch 3, dadurch gekennzeichnet, daß man in an sich bekannter Weise 0,30 bis 0,40 Gew.-% Nitroglycerin, 24,60 bis 24,70 Gew.-% Ethanol, 32,00 Gew.-% 1.2-Propylenglykol und 43,00 Gew.-% gereinigtes Wasser mischt, die erhaltene Lösung auf den pH-Wert 6,0 einstellt und in geeignete Primärpackmittel verbringt.

## Claims

1. A hydrophilic aqueous pump spray composition comprising nitroglycerin and one or more customary adjuvants particularly with a content of ethanol, characterized in that the composition comprises 0.15 to 0.50 weight/% of nitroglycerin, 24.50 to 24.85 weight/% of ethanol, 32.00 weight/% of 1,2-propyleneglycol and 43.00 weight/% of purified water and is adjusted to a pH value of 3.0 to 6.0.

2. A pump spray composition according to claim 1 characterized in that the composition comprises 0.30 to 0.40 weight/% nitroglycerin and 24.60 to 24.70 weight/% ethanol and is adjusted to a pH value of 6.0.

3. Method for the manufacture of a hydrophilic aqueous pump spray composition containing nitroglycerin and one or more customary adjuvants according to claims 1 and 2, characterized in that in an usual manner are mixing together 0.15 to 0.50 weight/% of nitroglycerin, 24.50 to 24.85 weight/% of ethanol, 32.00 weight/% of 1,2-propyleneglycol and 43.00 weight/% of purified water to form a solution which is then adjusted to a pH value of 3.0 to 6.0 and is filled into a suitable primary packaging means.

4. Method for the manufacture of a hydrophilic aqueous pump spray composition containing nitroglycerin and one or more customary adjuvants according to claim 3 characterized in that in an usual manner are mixing together 0.30 to 0.40 weight/% of nitroglycerin, 24.60 to 24.70 weight/% of ethanol, 32.00 weight/% of 1,2-propyleneglycol and 43.00 weight/% of purified water to form a solution which is then adjusted to a pH value of 6.0 and is filled into a suitable primary packaging means.

## Revendications

1. Préparation de spray à pompe aqueux hydrophile contenant de la nitroglycérine et un ou plusieurs adjuvants usuels et, en particulier, contenant de l'éthanol, caractérisée en ce que la préparation contient de 0,15 à 0,50% en poids de nitroglycérine, de 24,50 à 24,85% en poids d'éthanol, de 32% en poids de 1,2-prolypropylèneglycol et de 43% en poids d'eau purifiée et que le pH est réglé a une valeur de 3 à 6.

2. Préparation de spray à pompe selon la revendication 1, caractérisée en ce que la préparation contient de 0,30% à 0,40% en poids de nitroglycérine et de 24,60 à 24,70% en poids d'éthanol et que le pH est réglé à une valeur égale à 6.

3. Procédé de préparation d'une préparation de spray à pompe aqueux hydrophile contenant de la nitroglycérine et un ou plusieurs adjuvants usuels selon les revendications 1 et 2, caractérisé en ce que l'on mélange, d'une manière connue en soi, de 0,15 à 0,50% en poids de nitroglycérine, de 24,50 à 24,85% en poids d'éthanol, de 32% en poids de 1,2-propylèneglycol et de 43% en poids d'eau purifiée, que l'on règle le pH de la solution obtenue entre 3 et 6 et que l'on introduit cette solution dans un emballagae primaire approprié.

4. Procédé de préparation d'une préparation de spray à pompe aqueux hydrophile contenant de la nitroglycérine et un ou plusieurs adjuvants usuels selon la revendication 3, caractérisé en ce que l'on mélange, d'une manière connue en soi, de 0,30% à 0,40% en poids de nitroglycérine, de 24,60 à 24,70% en poids d'éthanol, de 32% en poids de 1,2-propylèneglycol et de 43% en poids d'eau purifiée, que l'on règle le pH de la solution obtenue à une valeur égale à 6 et que l'on introduit cette solution dans un emballage primaire approprié.
